(19) Europäisches Patentamt
European Patent Office
Office européen des brevets

(11) EP 3 864 966 A1

(12) EUROPÄISCHE PATENTANMELDUNG

(43) Veröffentlichungstag:
**18.08.2021 Patentblatt 2021/33**

(51) Int Cl.:
***A23L 2/60*** (2006.01) ***A23L 27/30*** (2016.01)
***A23L 29/30*** (2016.01) ***A61Q 11/00*** (2006.01)

(21) Anmeldenummer: **20156808.6**

(22) Anmeldetag: **12.02.2020**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**KH MA MD TN**

(71) Anmelder: **Savanna Ingredients GmbH**
**50189 Elsdorf (DE)**

(72) Erfinder:
- **BACHMANN, Vanessa**
**41065 Mönchengladbach (DE)**
- **KLEIN, Carolin Ellen**
**50389 Wesseling (DE)**
- **VEHLOW, Nathalie**
**50226 Frechen (DE)**
- **KAUFMANN, Birgit**
**50933 Köln (DE)**
- **KOCH, Timo**
**50189 Elsdorf (DE)**

(74) Vertreter: **Fabry, Bernd**
**IP2 Patentanwalts GmbH**
**Schlossstrasse 523**
**41238 Mönchengladbach (DE)**

# (54) ORALE ZUBEREITUNGEN MIT KRISTALLINER ALLULOSE

(57) Vorgeschlagen werden orale Zubereitungen, nämlich Nahrungsmittel, Mund- und Zahnpflegemittel sowie pharmazeutische Mittel enthaltend Allulose in kristalliner Form.

EP 3 864 966 A1

**Beschreibung**

## GEBIET DER ERFINDUNG

Die Erfindung befindet sich auf dem Gebiet der Lebensmitteltechnologie und betrifft orale Zubereitungen, insbesondere Nahrungsmittel, Mund- und Zahnpflegemittel und pharmazeutische Zubereitungen, die einen Gehalt an Allulose in kristalliner Form, aufweisen.

## TECHNOLOGISCHER HINTERGRUND

Aufgrund des wachsenden Interesses eines großen Teils der Bevölkerung an *"gesunder Ernährung"* und gesunder Lebensweise allgemein, haben kalorienfreie oder zumindest kalorienarme Zucker und Zuckerersatzstoffe ein großes Interesse in der Lebensmittelindustrie und in der wissenschaftlichen Community erweckt.

Zu den wichtigsten natürlichen Zuckerersatzstoffen gehören Stevia, Erythrit, Xylit, Sucralose und Sorbit. Die Süßkraft dieser Stoffe im Vergleich zu Saccharose liegt zwischen 0,5 und 600, die Kalorienzahl zwischen 0 und 260. Alle diese Stoffe weisen jedoch Nachteile auf, zum Beispiel:

- Stevia hat einen intensiven unangenehmen metallischen Eigengeschmack und ist zum Backen ungeeignet;
- Erythrit und Xylit lassen sich weder karamellisieren noch bräunen (Maillard-Reaktion);
- Sucralose ist für Menschen mit Fructoseintoleranz ungeeignet;
- Sorbit ist für viele Menschen, insbesondere für Diabetiker unverträglich.

Aber auch bei den konfektionierten Produkten gibt es Raum für Verbesserungen, angefangen bei der zu verbessernden Haltbarkeit von Nahrungsmitteln im Allgemeinen oder speziell die mangelnde Farbbeständigkeit von Tomatenketchup, die nicht zufriedenstellende Löslichkeit vieler Instantgetränke, speziell im Automatenbereich, bis hin zu sensorischen Effekten wie der mangelnden Cremigkeit von Speiseeis, Joghurt oder Frischkäse, um nur einige exemplarischen Mängel zu nennen.

## RELEVANTER STAND DER TECHNIK

Verschiedene Verfahren zur Herstellung von Allulose sind aus dem Stand der Technik bekannt, wie beispielweise aus den Dokumenten WO 2018/087261 A1 (Pfeifer & Langen GmbH & Co. KG) oder WO 2016/135358 A1 (Tate & Lyle Technology Limited).

WO 2018 20103 A1 (GIVAUDAN) beansprucht eine Sweetener-Mischung aus Stevia und weiteren Süßstoffen wie z.B. Psicose. Psicose steht dabei aber nicht im Vordergrund, sondern Mogroside.

WO 2014 186084 A1 (PEPSICO) beansprucht eine Getränkezusammensetzung, die als Süßstoffkomponente Erythrol, Psicose und Rebaudiosid M enthält.

## AUFGABE DER ERFINDUNG

Die Aufgabe der vorliegenden Erfindung hat somit darin bestanden, einen Ersatzstoff für Haushaltszucker zu finden, der zum einen entweder kalorienfrei ist oder im direkten Vergleich zumindest deutlich weniger Kalorien aufweist und anders als andere Zuckerersatzstoffe aus dem Stand der Technik keinen Anlass zu sensorischen oder geschmacklichen Beanstandungen gibt, die Haltbarkeit von Nahrungsmitteln verlängert, in Abmischung mit diesen anderen Zuckerersatzstoffen deren sensorischen oder geschmacklichen Nachteile ganz oder zumindest überwiegend ausgleicht und sensorische, geschmackliche und/oder physikalische Nachteile, die sich für die Endformulierungen ergeben ausräumen.

## BESCHREIBUNG DER ERFINDUNG

Gegenstand der vorliegenden Erfindung sind orale Zubereitungen, enthaltend Allulose in kristalliner Form.

Überraschenderweise wurde gefunden, dass der Austausch von Haushaltszucker gegen Allulosekristalle, einen positiven Einfluss sowohl auf die sensorischen als auch physikalischen und geschmacklichen Eigenschaften der oralen Zubereitungen hat, die im Folgenden näher beschrieben werden.

Insbesondere wurde gefunden, dass beim Austausch von Haushaltszucker gegen Allulose der $a_w$-Wert der

konfektionierten Produkte gesenkt wird. Mit diesem Wert wird die Wasseraktivität eines Lebensmittels beschrieben. Er stellt ein Maß für das "verfügbare" oder "aktive" Wasser im Gegensatz zur bloßen Angabe des Wassergehalts dar. Die Bedeutung dieser Größe ergibt sich daraus, dass für die Haltbarkeit von Lebensmitteln nicht nur der reine Wassergehalt von Bedeutung ist, sondern auch, in welchem Maße das Wasser durch das Substrat gebunden ist. Die Wasseraktivität beeinflusst das Wachstum von Mikroorganismen, den Ablauf chemischer Prozesse wie Fettoxidation und nichtenzymatische Bräunung, die Aktivität von Enzymen, und die physischen Eigenschaften des Lebensmittels. Die Wasseraktivität ist definiert als Verhältnis des Wasserdampfpartialdrucks in dem Lebensmittel (p) zum Sättigungsdampfdruck von reinem Wasser ($p_0$) bei einer bestimmten Temperatur:

$$a_w = p/P_0$$

Die Wasseraktivität ist gleichbedeutend mit der (relativen) Gleichgewichtsfeuchtigkeit (RGF), das heißt der relativen Luftfeuchtigkeit, bei der das Lebensmittel (wiederum bei der gegebenen Temperatur) mit der Umgebungsluft im Gleichgewicht steht, also weder Wasser verliert noch aufnimmt. Allerdings wird die relative Luftfeuchtigkeit meistens in der Hilfsmaßeinheit Prozent angegeben, so dass man die relative Gleichgewichtsfeuchtigkeit berechnet als:

$$RGF = a_w * 100$$

Gleichzeitig entspricht also die Wasseraktivität ($a_w$) 0-1 einer relativen Luftfeuchte von 0-100 %. Die Messung der Wasseraktivität erfolgt im einfachsten Fall, indem eine Probe des Lebensmittels in einen hermetisch verschlossenen Behälter gebracht und mit einem Hygrometer die Luftfeuchtigkeit gemessen wird, die sich in dem Behälter einstellt.

Die oralen Zubereitungen können die kristalline Allulose in Mengen von etwa 0,1 bis etwa 50 Gew.-%, vorzugsweise von etwa 0,5 bis etwa 35 Gew.-% und insbesondere von etwa 1 bis etwa 10 Gew.-% enthalten.

Die oralen Zubereitungen, die den Gegenstand der vorliegenden Erfindung darstellen, sind dem Grunde nach bekannt. Die Erfindung besteht jedoch darin, den darin enthaltenen Haushaltszucker ganz oder teilweise gegen kristalline Allulose auszutauschen. Ein teilweiser Austausch bedeutet, dass mindestens 10 und maximal 90 Gew.-%, vorzugsweise etwa 20 bis etwa 90 Gew.-%, weiter bevorzugt etwa 30 bis etwa 80 Gew.-% und insbesondere etwa 50 bis etwa 70 Gew.-% Haushaltszucker (oder gegebenenfalls andere Kohlenhydrate) gegen kristalline Allulose ausgetauscht werden.

## ALLULOSE IN KRISTALLINER FORM

Allulose (Psicose) ist ein kalorienarmer Zucker mit einem ähnlichen süßen Geschmack von Zucker. Allulose ist einer von vielen verschiedenen Zuckern, die in der Natur in sehr geringen Mengen vorkommen. Allulose wurde erstmals in Weizen identifiziert und seitdem in bestimmten Früchten wie Jackfrucht, Feigen und Rosinen gefunden. Allulose ist natürlich in kleinen Mengen in einer Vielzahl von süßen Lebensmitteln wie Karamellsauce, Ahornsirup und braunem Zucker enthalten. Allulose wird vom Körper absorbiert, aber nicht metabolisiert, sodass sie nahezu kalorienfrei ist.

Die Herstellung von Allulose bzw. Psicose aus Fructose ist seit 1995 bekannt, wie die Auswahl der folgenden Schriften zeigt:

- H. Itoh et al, Journal of Fermentation Bioengeneering, 80(1), 1995, S. 101-103 veröffentlicht die Herstellung von D-Psicose aus D-Fructose durch immobilisierte D-Tagatose 3-Epimerase.

- N. Wagner et al in Organic Process Research Development 2012, 16, S. 323-330 bezieht sich auf praktische Aspekte des integrierten Betriebs von Biotransformation und simulierter Bewegungsbetttrennung (SMB) für die feinchemische Synthese. D-Psicose wird unter Verwendung der D-Tagatose Epimerase-katalysierten Epimerisierung aus D-Fructose hergestellt.

- N. Wagner et al, in Chemical Engineering Science 137 (2015) S. 423-435 bezieht sich auf die modellbasierte Kostenoptimierung eines integrierten Prozesses zur enzymatischen Herstellung von Psicose bei erhöhten Temperaturen.

- N. Wagner et al, in Angewandte Chemie Int. Ed. Engl. 2015, 54(14), S. 4182-6 offenbart eine trennungsintegrierte Kaskadenreaktion zur Überwindung thermodynamischer Einschränkungen bei der Seltenzucker-Synthese.

- Bosshart et al, in Biotechnology Bioengineering 2016, 113(2), S. 349-58 bezieht sich auf die Produktion der seltenen

Zucker D-Psicose und L-Tagatose durch zwei künstlich hergestellte D-Tagatose-Epimerasen.

- N. Wagner, et al., in Journal of Chromatography A 2015, 1398, S. 47-56 offenbart ein Verfahren zur wirtschaftlichen Herstellung von d-Psicose durch simulierte Wanderbettchromatographie.
- Gegenstand der WO 2018 087261 A1 (PFEIFER&LANGEN) ist ein Verfahren zur Synthese eines Einfachzuckers, vorzugsweise von D-Allulose aus einem Edukt, vorzugsweise von D-Fructose unter heterogener oder homogener Katalyse, die eine chemische und/oder enzymatische Katalyse beinhaltet, wobei die Synthese in mindestens zwei in Reihe geschalteten Reaktoren durchgeführt wird und das aus dem ersten Reaktor austretende Reaktionsprodukt vor dem Eintritt in den zweiten Reaktor einer chromatographischen Trennung unterzogen wird. Vorzugsweise wird die chromatographische Trennung in ein simuliertes Wanderbett integriert.

In der Regel wird Allulose in Form eines Sirups kommerzialisiert, der nach einem der oben genannten Verfahren erhältlich ist. In einer bevorzugten Ausführungsform umfasst oder besteht der flüssige Allulose-Sirup aus folgenden Komponenten, bezogen auf die im Sirup enthaltene Trockenmasse:

(i) etwa 90 bis etwa 95 Gew.-% Allulose,

(ii) etwa 5 bis etwa 10 Gew.-% Fructose,

(iii) 0 bis etwa 5 Gew.-% weitere Kohlenhydrate, verschieden von Allulose und Fructose,

mit der Maßgabe, dass sich die Mengenangaben zu 100 Gew.-% ergänzen. Der guten Ordnung halber sei darauf hingewiesen, dass Ausführungsformen, die sich zu mehr oder zu weniger als 100 Gew.-% ergeben, weder von der Erfindung eingeschlossen noch von den Ansprüchen umfasst werden. Der Fachmann ist in jedem Fall in der Lage, nach Maßgabe von Beschreibung und Beispielen solche Zusammensetzungen aufzufinden, die die technische Lehre erfüllen, ohne dazu erfinderisch tätig werden zu müssen.

Der Allulose-Sirup wird anschließend in einem ein- oder mehrstufigen Verfahren aufkonzentriert, bis er etwa 80 bis 90 Gew.-% Trockenmasse aufweist und dann kristallisiert. Die resultierenden Allulosekristalle weisen typisch einen Fructosegehalt von maximal 1,5 Gew.-% (vorzugsweise 0,5 bis 1 Gew.-%) und einen Aschegehalt von weniger als 0,1 Gew.-% auf.

In einer bevorzugten Ausführungsform der vorliegenden Erfindung wird kristalline Allulose eingesetzt, die sich durch eine konkrete durchschnittliche Korngrößenverteilung auszeichnet. Mit solchen Produkten lassen sich die besten anwendungstechnischen und sensorischen Ergebnisse erzielen. Entsprechende Produkte lassen sich beispielsweise nach dem folgenden Verfahren erhalten:

(a) Bereitstellen einer wässrigen Allulose-Zubereitung, vorzugsweise einem Verdampferkonzentrat ("Allulose-Sirup");

(b) Aufkonzentrieren der Zubereitung aus Schritt (a) bis die Sättigungskonzentration überschritten wird;

(c) Versetzen der übersättigten Zubereitung aus Schritt (c) mit Allulose-Impfkristallen; und

(d) Abkühlen der Zubereitung aus Schritt (c) mit einer Geschwindigkeit von 0,1 bis etwa 1 K/h bis ein Kristallgehalt von etwa 30 bis etwa 50 Gew.-% erreicht wird, sowie

(e) Abtrennen und gegebenenfalls Trocknen der Kristalle.

Die besagte bevorzugte Korngrößenverteilung wurde mittels dynamischer Bildanalyse (Camsizer X2 der Firma Retsch mit X-Jet Modul) ermittelt, wobei ein Dispergierdruck von 5, 20 und 460 kPa angelegt wurde. Die Auswertung erfolgte in drei Größenmodellen, nämlich:

(I) $X_{c(min)}$: zeigt die Partikelbreite an, welche aus der schmalsten aller gemessenen Sehnen $X_c$ bestimmt wird

(II) $X_{Fe(max)}$: zeigt die Partikellänge an, welche aus dem längsten aller gemessenen Feret-Durchmesser $X_{Fe}$ bestimmt wird.

(III) $X_{area}$ : zeigt den äquivalenten Partikeldurchmesser an, welcher dem Durchmesser eines flächengleichen Kreises

entspricht

Die bevorzugten Korngrößenverteilungen sind in der folgenden Tabelle wiedergegeben; der Wert "b/l d50" gibt jeweils das Breiten/Längenverhältnis der Kristalle beim d50 wieder. Es ist besonders bevorzugt, wenn dieser Wert zwischen 0,6 und 1,0, insbesondere zwischen 0,7 und 0,9 und optimal zwischen 0,75 und 0,8 liegt.

| **Parameter** | **Dispergierdruck [kPa]** | **Methode I** | **Methode II** | **Methode II** |
|---|---|---|---|---|
| Minimum d10 [μm] | 5 | > 70 | > 100 | >85 |
| Minimum d50 [μm] | 5 | > 140 | > 220 | > 185 |
| Minimum d90[μm] | 5 | > 230 | > 340 | > 265 |
| b/l d50 | 5 | 0,52-1,0 | 0,52-1,0 | 0,52-1,0 |
| Minimum d10 [μm] | 20 | > 50 | > 70 | > 65 |
| Minimum d50 [μm] | 20 | > 120 | > 200 | > 180 |
| Minimum d90[μm] | 20 | > 220 | > 330 | > 260 |
| b/l d50 | 20 | 0,53-1,0 | 0,55-1,0 | 0,55-1,0 |
| Minimum d10 [μm] | 460 | > 10 | > 8 | > 10 |
| Minimum d50 [μm] | 460 | > 40 | > 65 | > 50 |
| Minimum d90[μm] | 460 | > 130 | > 170 | > 120 |
| b/l d50 | 460 | 0,60 - 1,0 | 0,65-1,0 | 0,65-1,0 |

Im Sinne der vorliegenden Erfindung lassen sich die bevorzugten Allulosekristalle durch eine, zwei oder alle drei genannten Verfahren zur Korngrößenverteilung definieren.

## NAHRUNGSMITTEL

In einer ersten Ausführungsform der vorliegenden Erfindung kann es sich bei den oralen Zubereitungen um Nahrungsmittel handeln. In Betracht kommen dabei beispielsweise und nicht abschließend:

- Getränke,
- Backwaren,
- Getreideprodukte,
- Knabberartikel,
- Süßwaren,
- Milchprodukte,
- Fruchtzubereitungen,
- Würzmittel,
- Gemüsezubereitungen,
- Fleischwaren oder
- Gewürze und Gewürzmischungen.

Die Gruppe der **Getränke** umfasst sowohl alkoholische als auch nicht-alkoholische Produkte, wie beispielsweise

Kaffee, Tee, Eistee, Wein, weinhaltige Getränke, Bier, bierhaltige Getränke, Liköre, Schnäpse, Weinbrände, (karbonisierte) fruchthaltige Limonaden, (karbonisierte) isotonische Getränke und mit Geschmacksstoffen angereichertes Mineralwasser, (karbonisierte) Erfrischungsgetränke, Nektare, Schorlen, Obst- und Gemüsesäfte, Frucht- oder Gemüsesaftzubereitungen, Instantgetränke, wie beispielsweise Instant-Kakao-Getränke, Instant-Tee-Getränke, Instant-Kaffeegetränke oder Instant-Fruchtgetränke. Erfindungsgemäße Getränke mit kristalliner Allulose weisen beispielsweise die folgenden Vorteile auf:

- verbesserte Fruchtigkeit;
- keine negative Beeinflussung der Schaumstabilität;
- verbessertes Mundgefühl im Gegensatz zu anderen kalorienreduzierten Getränken;
- Süßung ohne Kalorien und ohne E-Nummer;
- echter Zucker ohne Kalorien + ohne "Off-taste";
- 100% Saccharose-Austausch technisch möglich;
- geeignet für Diabetiker;
- geeignet für sämtliche Formate (von Instant bis Biermisch);
- transparente Farbe im Endprodukt;
- hervorragende Synergien in Kombination mit Saccharose (Sensorik, Haptik, Optik);
- Saccharose-ähnliches Süßeprofil;
- erhöhte Farbintensität, z.B. bei Orangenlimonade;
- verbesserte Farbstabilität;
- verbessertes $CO_2$-Haltevermögen nach dem Öffnen der Flasche;
- verbesserte Spritzigkeit und Frische;
- verbessertes Mundgefühls; sowie
- durch schnellere Löslichkeit sehr gute Eignung für Automatengetränke (Instant).

Ebenfalls geeignet sind **Backwaren, Getreideprodukte und Knabberartikel.** Hierzu zählen z.B. Brot, Trockenkekse, Kuchen, Muffins, Donuts und sonstiges Gebäck. Als Knabberartikel kommen beispielsweise gebackene oder frittierte Kartoffelchips oder Kartoffelteigprodukte, Extrudate auf Mais- oder Erdnussbasis in Frage. Als Getreideprodukte sind beispielsweise Frühstückscerealien, Müsliriegel, vorgegarte Fertigreis-Produkte zu nennen.
Als **Süßwaren** kommen beispielsweise Schokoladen, Schokoladenriegelprodukte, sonstige Riegelprodukte, Fruchtgummi, Gummibonbons, Hart- und Weichkaramellen, Kaugummi, Tabletten, Mints sowie Überzüge (Fondants) in Betracht.
Erfindungsgemäße Produkte aus diesen Gruppen, die Allulose-Kristalle enthalten, weisen mindestens einen der folgenden Vorteile auf:

- Süßung ohne Kalorien und ohne E-Nummer
- echter Zucker ohne Kalorien + ohne "Off-taste"
- geeignet als Feuchthaltemittel; verhindert Austrocknen, verlängert das Mindesthaltbarkeitsdatum;
- macht Produkt geschmeidiger/weicher, trocknet Produkt nicht aus wie andere Zuckerersatzstoffe
- verbessert Aroma und Fruchtigkeit von Zuckersäurecoatings (Bsp. Fuchtgummis)
- verbesserte Löslichkeit bei Komprimaten und Brausetabletten
- geeignet in Riegeln (Müsli, Proteinriegel)

- verbessertes sensorisches Profil durch Karamellnote;
- weichere und saftigere Krume im Vergleich zu anderen Zuckern;
- verbesserte Viskosität des Teiges;
- 100% Saccharose-Austausch möglich;
- geeignet für Diabetiker;
- verringerte Backzeit durch erhöhte Reaktivität der Allulose-Kristalle;
- optimales Bräunungsergebnis bei verringerten Backtemperaturen, weniger oder gar kein Zusatz von Farbstoffen erforderlich;
- bessere Verarbeitungsfähigkeiten von Backmischungen;
- Verstärkung des Umami-Geschmacks bei Snacks;
- Verstärkung der Karamellnote bei Toffees sowie
- Verstärkung des Schokoladengeschmacks und/oder der Kakaonote bei Schokoladen sowie schoko- oder kakaohaltigen Produkten.

Als **Milchprodukte** kommen beispielsweise Milchgetränke, Buttermilchgetränke, Milcheis, Joghurt, Kefir, Frischkäse, Weichkäse, Hartkäse, Trockenmilchpulver, Molke, Molkegetränke, Butter, Buttermilch, teilweise oder ganz hydrolysierte Milchprotein-haltige Produkte), Produkte aus Sojaprotein oder anderen Sojabohnen-Fraktionen (beispielsweise Sojamilch und daraus gefertigte Produkte, Fruchtgetränke mit Sojaprotein, Sojalecithin-haltige Zubereitungen, fermentierte Produkte wie Tofu oder Tempe oder daraus gefertigte Produkte), Produkte aus anderen pflanzlichen Proteinquellen, beispielsweise Haferprotein-Getränke in Frage. Erfindungsgemäße Milchprodukte, die kristalline Allulose enthalten, weisen beispielsweise die folgenden Vorteile auf:

- Süßung ohne Kalorien und ohne E-Nummer;
- verstärkte Süßewahrnehmung in aromatisierten Milchgetränken durch Kombination von kristalliner Allulose und Saccharose (Maillard-Reaktion) in Einsatzmengen von 2-15 Gew.-%;
- positive Geschmacksverstärkung in Gegenwart von Proteinen;
- echter Zucker ohne Kalorien + ohne "Off-taste";
- 100% Saccharose-Austausch technisch möglich;
- geeignet für Diabetiker;
- verbessertes Geschmacksprofil (z.B. Karamelleis: Kein oder verringerter Aromazusatz);
- Senkung des Gefrierpunktes und verbessertes Schmelzverhalten bei Speiseeis;
- weichere, cremigere Textur bei Joghurt und Quarkzubereitungen, Desserts und Cream-Cheese;
- erhöhtes sensorisches Kälteempfinden;
- intensivierte Fruchtigkeit in Sorbets; sowie
- Verringerung des negativen Kochgeschmack bei gängigen Erhitzungsverfahren.

Bei den **Fruchtzubereitungen** sind beispielsweise Konfitüren, Fruchteis, Fruchtsoßen, Fruchtfüllungen zu nennen. Auch Honig und honigartige Produkte, wie Ahornsirup werden im Sinne der Erfindung hierunter verstanden. Erfindungsgemäße Produkte, die in diese Gruppe fallen, zeigen beispielsweise die folgenden Vorteile:

- Süßung ohne Kalorien und ohne E-Nummer;
- echter Zucker ohne Kalorien + ohne "Off-taste";
- 100% Saccharoseaustausch sowie beliebige Kombinationen möglich;
- geeignet für Diabetiker;

- verbessertes Geschmacksprofil;
- intensivere Farbgebung;
- verlängerte Haltbarkeit durch niedrigeren aw-Wert
- Verstärkung der Geschmacksintensität (z.B. intensivere Fruchtigkeit);
- bessere Streichfähigkeit bei Fruchtaufstrichen;
- bessere Cremigkeit bei Honig und honigartigen Produkten;
- bessere Einarbeitung von Fruchtzubereitungen in Joghurt und Quarkzubereitungen, Desserts;
- gleichbleibende Fruchtigkeit bei geringerem Säurezusatz;
- geringere Schaumbildung; sowie
- Herstellung von Gelierzucker ohne Trocknung möglich.

Als **Würzmittel und Gemüsezubereitungen** eignen sich beispielsweise Ketchup (speziell Gewürz-, Curry- und BBQ-Ketchup), Soßen, Trockengemüse, Tiefkühlgemüse, vorgegarte Gemüse, eingekochte Gemüse. Hierzu zählen auch Produkte auf Fett- und Ölbasis oder Emulsionen derselben (beispielsweise Mayonnaise, Remoulade, Dressings), sonstige Fertiggerichte und Suppen (beispielsweise Trockensuppen, Instant-Suppen, vorgegarte Suppen), Gewürze, Würzmischungen sowie insbesondere Aufstreuwürzen (englisch: Seasonings), die beispielsweise im Snackbereich Anwendung finden. Erfindungsgemäße Produkte aus dieser Gruppe zeigen beispielsweise die folgenden Vorteile:

- Süßung ohne Kalorien und ohne E-Nummer;
- echter Zucker ohne Kalorien und ohne "Off-taste";
- 100% Saccharose-Austausch möglich;
- geeignet für Diabetiker;
- verbesserte Farbgebung;
- verlängerte Haltbarkeit;
- Verstärkung der Geschmacksintensität, z.B. intensivere Fruchtigkeit der Tomate in Ketchup);
- verbesserte Viskosität von Ketchup; sowie
- Verstärkung des Umamigeschmacks.
- Gute stabilisierende Eigenschaften in Lebensmittelemulsionen

Zu den geeigneten **Fleischwaren** gehören beispielsweise Schinken, Frischwurst- oder Rohwurstzubereitungen, gewürzte oder marinierte Frisch- oder Pökelfleischprodukte.

Ebenfalls umfasst die Gruppe **Gewürze und Gewürzmischungen** sowie Süßkaftverstärkern, Geschmacksverstärker und Maskierungsmittel, gegebenenfalls in verkapselter Form.

**Süßstoffe,** deren Süßkraft verstärkt und/oder deren unangenehmen Geschmacksnoten (adstringierend, metallisch, teerig, fettig) überdeckt oder maskiert werden, umfassen beispielsweise: Sucrose/Saccharose, Trehalose, Lactose, Maltose, Melizitose, Raffinose, Palatinose, Lactulose, D-Fructose, D-Glucose, D-Galactose, L-Rhamnose, D-Sorbose, D-Mannose, D-Tagatose, D-Arabinose, L-Arabinose, D-Ribose, D-Glycerinaldehyd, oder Maltodextrin. Ebenfalls geeignet sind pflanzliche Zubereitungen, die diese Stoffe enthalten, beispielsweise auf Basis von Zuckerrüben (Beta vulgaris ssp., Zuckerfraktionen, Zuckersirup, Melasse), Zuckerrohr (Saccharum officinarum ssp., Melasse, Zuckerrohrsirup), Ahornsirup (Acer ssp.) oder Agaven (Agavendicksaft).

In Betracht kommen auch synthetische, d.h. in der Regel enzymatisch hergestellte Stärke oder Zuckerhydrolysate (Invertzucker, Fructosesirup);

- Fruchtkonzentrate (z.B. auf Basis von Äpfeln oder Birnen);
- Zuckeralkohole (z.B. Erythritol, Threitol, Arabitol, Ribotol, Xylitol, Sorbitol, Mannitol, Dulcitol, Lactitol);
- Proteine (z.B. Miraculin, Monellin, Thaumatin, Curculin, Brazzein);
- Süßstoffe (z.B. Magap, Natriumcyclamat, Acesulfam K, Neohesperidin Dihydrochalcon, Saccharin Natriumsalz, Aspartam, Superaspartam, Neotam, Alitam, Sucralose, Stevioside, Rebaudioside, Lugduname, Carrelame, Sucrononate, Sucrooctate, Monatin, Phenylodulcin);
- Süß schmeckende Aminosäuren (z.B. Glycin, D-Leucin, D-Threonin, D-Asparagin, D-Phenylalanin, D-tryptophn, L-Prolin);
- Weitere süß schmeckende niedermolekulare Substanzen, wie z.B. Hernandulcin, Dihydrochalconglykoside, Glycyrrhizin, Glycerrhetinsäure, ihre Derivate und Salze, Extrakte von Lakritz (Glycyrrhizza glabra ssp.), Lippia dulcis Extrakte, Momordica ssp. Extrakte oder

Einzelsubstanzen wie z.B. Momordica grosvenori [Luo Han Guo] und die daraus gewonnenen Mogroside, Hydrangea dulcis oder Stevia ssp. (z.B. Stevia rebaudiana) Extrakte, wie sie nachfolgend noch näher erläutert werden.

Rebaudioside gehören zu den Steviosiden, den Hauptbestandteilen der Pflanze *Stevia rebaudiana,* die auch als Süßkraut oder Honigkraut bezeichnet wird.

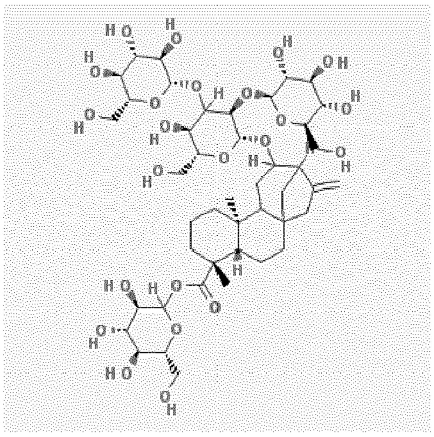

Rebaudiosid

10 % der Trockenmasse der Blätter macht das Diterpenglykoid Steviosid aus, gefolgt von Rebaudiosid A (2 bis 4 Gew.-%) sowie zehn weiteren Steviolglycosiden, wie etwa dem Dulcosid. Rebaudioside und Stevia-Extrakt sind als Süßungsmitteln inzwischen in den meisten Staaten zugelassen; eine tägliche Aufnahmemenge von bis zu 4 mg Steviosid pro Kilogramm Körpergewicht wird als unbedenklich angesehen. Im Sinne der Erfindung können einzelne Rebaudioside oder die Extrakte der Stevia-Pflanze eingesetzt werden. Besonders bevorzugt ist jedoch die Verwendung von Rebaudiosid A, da dieser Stoff eine geringere Bitterkeit und die höchste Süßkraft aufweist.

Alternativ oder ergänzend zu den Rebaudiosiden bzw. Stevia Extrakten können als Süßstoffe auch verschiedene andere Wirkstoffe eingesetzt werden; es sind dies:

- Naringin,
- Dihydrochalcone,
- Mogroside sowie
- Extrakten der Pflanzen der Gattung *Rubus.*

Naringin ist ein polyphenolisches Glykosid, das in der Grapefruit und dem Pomelo enthalten ist und diesen einen bitteren Geschmack verleiht. Der Stoff ist insbesondere wegen seiner lipidsenkenden Wirkung bekannt.

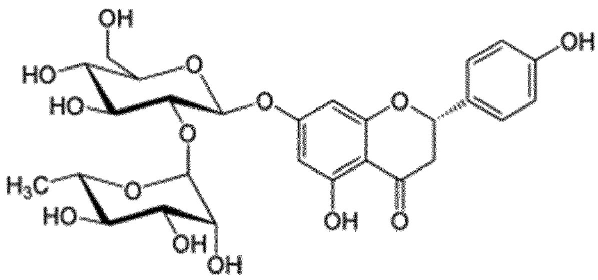

Naringin

Auch die Dihydrochalcone stellen Polyphenole dar, wobei insbesondere die beiden Vertreter Dihydrochalcon und Neohesperidin Dihydrochalcon hervorzuheben sind, die als künstliche Süßungsmittel bekannt sind:

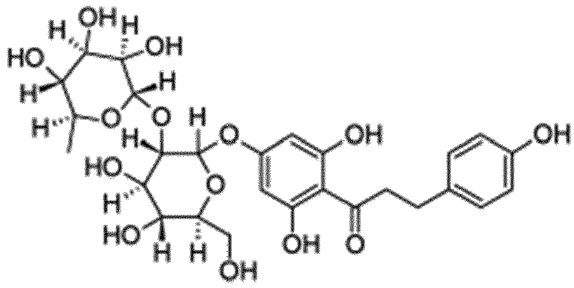

Dihydrochalcon

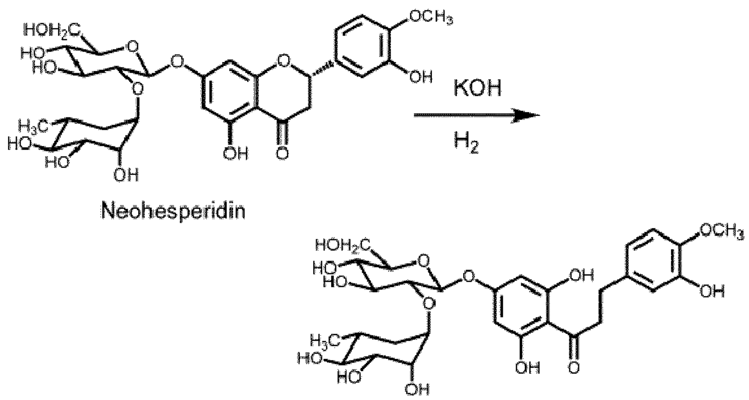


Neohesperidin

Neohesperidin dihydrochalcone

Unter der Bezeichnung Mogroside wird eine Gruppe von Glykosiden des Cucurbitans verstanden, die als Bestandteil des natürlichen Süßungsmittels Luo Han Guo bekannt sind. Hervorzuheben ist hier das Mogrosid-5, das 400mal süßer als Zucker ist.

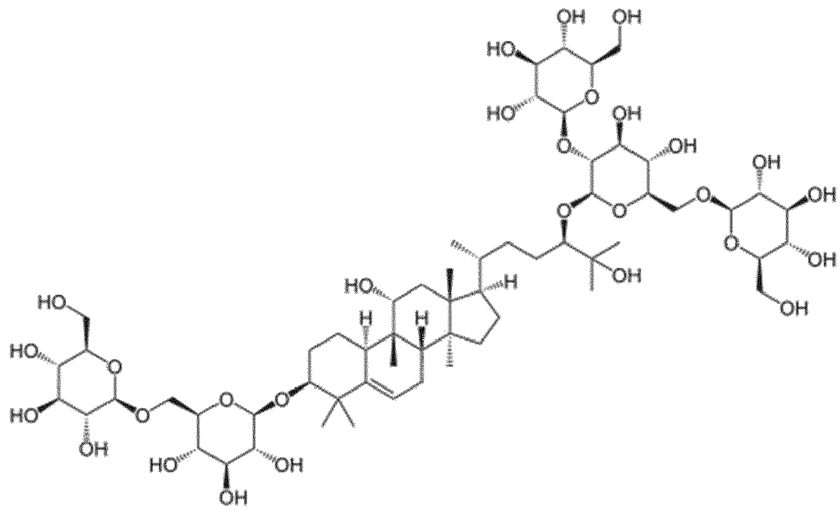

Mogrosid-5

Schließlich auch Extrakte der Pflanzen in Betracht, die ausgewählt sind aus der Gruppe, die gebildet wird von Rubus allegheniensis, Rubus arcticu, Rubus strigosus, Rubus armeniacus, Rubus caesius, Rubus chamaemorus, Rubus corylifolius agg., Rubus fruticosus agg., Rubus geoides, Rubus glaucus, Rubus gunnianus, Rubus idaeus, Rubus illecebrosus, Rubus laciniatus, Rubus leucodermis, Rubus loganobaccus, Rubus loxensis, Rubus nepalensis, Rubus nessensis, Rubus nivalis, Rubus odoratus, Rubus pentalobus, Rubus phoenicolasius, Rubus saxatilis, Rubus setchuenensis, Rubus spectabilis und Rubus ulmifolius sowie deren Gemischen. Hierbei handelt es sich im Wesentlichen um Extrakte von unterschiedlichen Brombeer- und Himbeerarten, die einen Gehalt an Rubosiden aufweisen. Bevorzugt sind Extrakte von Rubus Suavissimus.

Ein weiterer Wirkstoff in dieser Gruppe ist die Glycyrrhizinsäure oder ein entsprechendes Salz oder einen Extrakt, der diesen Stoff enthält.

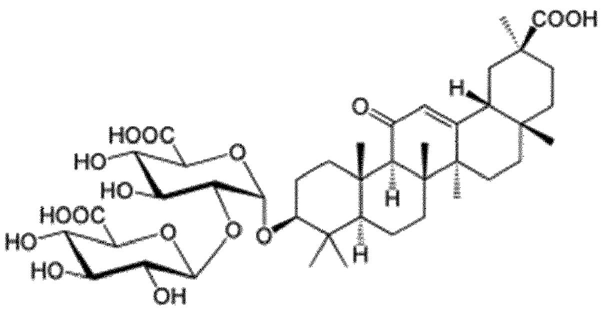

Glycyrrhizinsäure

Im Sinne der Erfindung ist es möglich, die Säure selbst, ihre Salze - beispielsweise Natrium-, Kalium- oder Ammoniumsalz - oder die Extrakte der Pflanze *Glycyrrhiza glabra* einzusetzen. Besonders bevorzugt ist das Monoammoniumglycyrrhizat.

**Geschmacksverstärker,** deren Wirkung zusätzlich verstärkt und/oder deren unangenehmen Geschmacksnoten (adstringierend, metallisch, teerig, fettig) überdeckt oder maskiert werden, umfassen beispielsweise: Nucleotide (z.B. Adenosin-5'-monophosphat, Cytidin-5'-monophosphat) oder deren pharmazeutisch akzeptable Salze, Lactisole, Natriumsalze (z.B. Natriumchlorid, Natriumlactat, Natriumcitrat, Natriumacetat, Natriumgluconoat), weitere Hydroxyflavanone (z.B. Eriodictyol, Homoeriodictyol oder deren Natriumsalze), insbesondere gemäß US 2002/0188019, Hydroxybenzoesäureamide nach DE 10 2004 041 496 (z.B. 2,4-Dihydroxybenzoesäurevanillylamid, 2,4-Dihydroxybenzoesäure-N-(4-hydroxy-3-methoxybenzyl)amid, 2,4,6-Trihydroxybenzoesäure-N-(4-hydroxy-3-methoxybenzyl)amid, 2-Hydroxy-benzoesäure-N-4-(hydroxy-3-methoxybenzyl)amid, 4-Hydroxybenzoesäure-N-(4-hydroxy-3-methoxybenzyl)amid, 2,4-Dihydroxybenzoesäure-N-(4-hydroxy-3-methoxybenzyl)amid-Mono-natriumsalz, 2,4-Dihydroxybenzoesäure-N-2-(4-hydroxy-3-methoxyphenyl)-ethylamid, 2,4-Dihydroxybenzoesäure-N-(4-hydroxy-3-ethoxybenzyl)amid, 2,4-Dihydroxybenzoesäure-N-(3,4-dihydroxybenzyl)amid und 2-Hydroxy-5-methoxy-N-[2-(4-hydroxy-3-methoxyphenyl)ethyl]amid (Aduncamid), 4-Hydroxybenzoesäurevanillylamid), bittermaskierende Hydroxydeoxybenzoine z.B. gemäß WO 2006/106023 (z.B. 2-(4-Hydroxy-3-methoxyphenyl)-1-(2,4,6-trihydroxyphenyl)ethanon, 1-(2,4-Dihydroxyphenyl)-2-(4-hydroxy-3-methoxyphenyl)ethanon, 1-(2-Hydroxy-4-methoxyphenyl)-2-(4-hydroxy-3-methoxyphenyl)ethanon), Aminosäuren (z.B. gamma-Aminobuttersäure nach WO 2005/096841 zur Verminderung oder Maskierung eines unangenehmen Geschmackseindrucks wie Bitterkeit), Äpfelsäureglycoside nach WO 2006/003107, salzig schmeckende Mischungen gemäß PCT/EP 2006/067120 Diacetyltrimere gemäß WO 2006/058893, Gemische von Molkeproteinen mit Lecithinen und/oder bittermaskierende Substanzen wie Gingerdione gemäß WO 2007/003527.

Bevorzugte Aromastoffe sind solche, die einen süßen Geruchseindruck verursachen, wobei der oder die weiteren Aromastoffe, die einen süßen Geruchseindruck verursachen, bevorzugt ausgewählt sind aus der Gruppe bestehend aus: Vanillin, Ethylvanillin, Ethylvanillinisobutyrat (= 3-Ethoxy-4-isobutyryloxybenzaldehyd), Furaneol® (2,5-Dimethyl-4-hydroxy-3(2H)-furanon) und Abkömmlinge (z.B. Homofuraneol, 2-Ethyl-4-hydroxy-5-methyl-3(2H)-furanon), Homofuronol (2-Ethyl-5-methyl-4-hydroxy-3(2H)-furanon und 5-Ethyl-2-methyl-4-hydroxy-3(2H)-furanon), Maltol und Abkömmlinge (z.B. Ethylmaltol), Cumarin und Abkömmlinge, gamma-Lactone (z.B. gamma-Undecalacton, gamma-Nonalacton), delta-Lactone (z.B. 4-Methyldeltalacton, Massoilacton, Deltadecalacton, Tuberolacton), Methylsorbat, Divanillin, 4-Hydroxy-2(oder 5)-ethyl-5(oder 2)-methyl-3(2H)furanon, 2-Hydroxy-3-methyl-2-cyclopentenone, 3-Hydroxy-4,5-dimethyl-2(5H)-furanon, Fruchtester und Fruchtlactone (z.B. Essigsäure-n-butylester, Essigsäureisoamylester, Propionsäureethylester, Buttersäureethylester, Buttersäure-n-butylester, Buttersäureisoamylester, 3-Methyl-buttersäureethylester, n-Hexansäureethylester, n-Hexansäure-allylester, n-Hexansäure-n-butylester, n-Octansäureethylester, Ethyl-3-methyl-3-phenylglycidat, Ethyl-2-trans-4-cis-decadienoat), 4-(p-Hydroxyphenyl)-2-butanon, 1,1-Dimethoxy-2,2,5-trimethyl-4-hexan, 2,6-Dimethyl-5-hepten-1-al, 4-Hydroxyzimtsäure, 4-Methoxy-3-hydroxyzimtsäure, 3-Methoxy-4-hydroxyzimtsäure, 2-Hydroxyzimtsäure, 2,4-Dihydroxybenzoesäure, 3-Hydroxybenzoesäure, 3,4-Dihydroxybenzoesäure, Vanillinsäure, Homovanillinsäure, Vanillomandelsäure und Phenylacetaldehyd.

Die Süßstoffe bzw. Geschmacksverstärker und die kristalline Allulose können dabei im Gewichtsverhältnis von etwa 10:90 bis 90:10, vorzugsweise von etwa 25:75 bis 75:25 und insbesondere etwa 40:60 bis 60:40 eingesetzt werden.

## MUND- UND ZAHNPFLEGEMITTEL

Erfindungsgemäße oral konsumierbare süß schmeckende Produkte können auch der Mund- und Zahnreinigung und-pflege dienen. Beispiele hierfür sind Zahnpasten, Zahngele, Zahnpulver, Mundwässer, (medizinische) Kaugummis und dergleichen. Erfindungsgemäße Produkte aus dieser Gruppe, weisen u.a. die folgenden Vorteile auf:

- Süßung ohne Kalorien und ohne E-Nummer;
- echter Zucker ohne Kalorien + ohne "Off-taste";
- 100% Saccharoseaustausch sowie beliebige Kombinationen möglich;
- geeignet für Diabetiker;
- verbessertes Geschmacksprofil;
- verbesserte Farbgebung;
- Verstärkung der Geschmacksintensität (z.B. intensiverer Minzgeschmack);
- Verstärkung der Kühlwirkung (z.B. länger anhaltender Mentholgeschmack); sowie
- geringere Schaumbildung.

Unter Zahnpasten oder Zahncremes werden im allgemeinen gelförmige oder pastöse Zubereitungen aus Wasser, Verdickungsmitteln, Feuchthaltemitteln, Schleif- oder Putzkörpern, Tensiden, Süßmitteln, Aromastoffen, deodorierenden Wirkstoffen sowie Wirkstoffen gegen Mund- und Zahnerkrankungen verstanden. In die erfindungsgemäßen Zahnpasten können alle üblichen Putzkörper, wie z. B. Kreide, Dicalciumphosphat, unlösliches Natriummetaphosphat, Aluminiumsilikate, Calciumpyrophosphat, feinteilige Kunstharze, Kieselsäuren, Aluminiumoxid und Aluminiumoxidtrihydrat eingesetzt werden.

Bevorzugt geeignete **Putzkörper** für die erfindungsgemäßen Zahnpasten sind vor allem feinteilige Xerogelkieselsäuren, Hydrogelkieselsäuren, Fällungskieselsäuren, Aluminiumoxid-trihydrat und feinteiliges alpha -Aluminiumoxid oder Mischungen dieser Putzkörper in Mengen von 15 bis 40 Gew.-% der Zahnpasta. Als Feuchthaltemittel kommen

vorwiegend niedermolekulare Polyethylenglykole, Glycerin, Sorbit oder Mischungen dieser Produkte in Mengen bis zu 50 Gew.-% in Frage. Unter den bekannten Verdickungsmitteln sind die verdickenden, feinteiligen Gelkieselsäuren und Hydrokolloide, wie z. B. Carboxymethylcellulose, Hydroxyethylcellulose, Hydroxypropylguar, Hydroxyethylstärke, Polyvinylpyrrolidon, hochmolekulares Polyethylenglykol, Pflanzengummen wie Traganth, Agar-Agar, Carragheenmoos, Gummi arabicum, Xantham-Gum und Carboxyvinylpolymere (z. B. Carbopol®-Typen) geeignet. Zusätzlich zu den Mischungen aus menthofuran und Mentholverbindungen können die Mund- und Zahnpflegemittel insbesondere oberflächenaktive Stoffe, bevorzugt anionische und nichtionische schaumstarke Tenside, wie die bereits oben genannten Stoffe, insbesondere aber Alkylethersulfat-Salze, Alkylpolyglucoside und deren Gemische.

Weitere übliche **Zahnpastenzusätze** sind:

- Konservierungsmittel und antimikrobielle Stoffe wie z. B. p- Hydroxybenzösäuremethyl-, -ethyl- oder -propylester, Natriumsorbat, Natriumbenzoat, Bromchlorophen, Phenylsalicylsäureester, Thymol und dergleichen;
- Antizahnsteinwirkstoffe, z. B. Organophosphate wie 1-Hydroxyethan- 1.1-diphosphonsäure, 1-Phosphonpropan-1,2,3-tricarbonsäure und andere, die z. B. aus US 3,488,419**,** DE 2224430 A1 und DE 2343196 A1 bekannt sind;
- andere karieshemmende Stoffe wie z. B. Natriumfluorid, Natriummonofluorphosphat, Zinnfluorid;
- Süssungsmittel, wie z. B. Saccharoseharin-Natrium, Natrium-Cyclamat, Sucrose, Lactose, Maltose, Fructose oder Apartam®, (L-Aspartyl- L-phenylalanin-methylester), Stivia-extrakte oder deren süßenden Bestandteile, insbesondere Ribeaudioside;
- Zusätzliche Aromen wie z. B. Eukalyptusöl, Anisöl, Fenchelöl, Kümmelöl, Methylacetat, Zimtaldehyd, Anethol, Vanillin, Thymol sowie Mischungen dieser und anderer natürlicher und synthetischer Aromen;
- Pigmente wie z. B. Titandioxid;
- Farbstoffe;
- Puffersubstanzen wie z. B. primäre, sekundäre oder tertiäre Alkaliphosphate oder Citronensäure/Natriumcitrat;

Wundheilende und entzündungshemmende Stoffe wie z. B. Allantoin, Harnstoff, Azulen, Kamillenwirkstoffe und Acetylsalicylsäurederivate.

Eine bevorzugte Ausführung der oralen Zubereitungen sind Zahnpasten in Form einer wässrigen, pastösen Dispersion, enthaltend Poliermittel, Feuchthaltemittel, Viskositätsregulatoren und gegebenenfalls weitere übliche Komponenten, sowie die Mischung aus Menthofuran und Mentholverbindungen in Mengen von 0,5 bis 2 Gew.-% enthalten.

In **Mundwässern** ist eine Kombination mit wässrig-alkoholischen Lösungen verschiedener Grädigkeit von ätherischen Ölen, Emulgatoren, adstringierenden und tonisierenden Drogenauszügen, zahnsteinhemmenden, antibakteriellen Zusätzen und Geschmackskorrigentien ohne weiteres möglich. Eine weitere bevorzugte Ausführung der Erfindung ist ein Mundwasser in Form einer wässrigen oder wässrig-alkoholischen Lösung enthaltend die Mischung aus Menthofuran und Mentholverbindungen in Mengen von 0,5 bis 2 Gew.-%. In Mundwässern, die vor der Anwendung verdünnt werden, können mit, entsprechend dem vorgesehenen Verdünnungsverhältnis, höheren Konzentrationen ausreichende Effekte erzielt werden.

Zur Verbesserung des Fließverhaltens können ferner **Hydrotrope,** wie beispielsweise Ethanol, Isopropylalkohol, oder Polyole eingesetzt werden; diese Stoffe entsprechen weitgehend den eingangs geschilderten Trägern. Polyole, die hier in Betracht kommen, besitzen vorzugsweise 2 bis 15 Kohlenstoffatome und mindestens zwei Hydroxylgruppen. Die Polyole können noch weitere funktionelle Gruppen, insbesondere Aminogruppen, enthalten bzw. mit Stickstoff modifiziert sein. Typische Beispiele sind

- Glycerin;
- Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
- technische Oligoglyceringemische mit einem Eigenkondensationsgrad von 1,5 bis 10 wie etwa technische Diglyceringemische mit einem Diglyceringehalt von 40 bis 50 Gew.-%;
- Methyolverbindungen, wie insbesondere Trimethylolethan, Trimethylolpropan, Trimethylolbutan, Pentaerythrit und

Dipentaerythrit;

- Niedrigalkylglucoside, insbesondere solche mit 1 bis 8 Kohlenstoffen im Alkylrest, wie beispielsweise Methyl- und Butylglucosid;
- Zuckeralkohole mit 5 bis 12 Kohlenstoffatomen, wie beispielsweise Sorbit oder Mannit,
- Zucker mit 5 bis 12 Kohlenstoffatomen, wie beispielsweise Glucose oder Saccharose;
- Aminozucker, wie beispielsweise Glucamin;
- Dialkoholamine, wie Diethanolamin oder 2-Amino-1,3-propandiol.

Als **Konservierungsmittel** eignen sich beispielsweise Phenoxyethanol, Formaldehydlösung, Parabene, Pentandiol oder Sorbinsäure sowie die unter der Bezeichnung Surfacine® bekannten Silberkomplexe und die in Anlage 6, Teil A und B der Kosmetikverordnung aufgeführten weiteren Stoffklassen.

Als **Parfümöle** seien genannt Gemische aus natürlichen und synthetischen Riechstoffen. Natürliche Riechstoffe sind Extrakte von Blüten (Lilie, Lavendel, Rosen, Jasmin, Neroli, Ylang-Ylang), Stengeln und Blättern (Geranium, Patchouli, Petitgrain), Früchten (Anis, Koriander, Kümmel, Wacholder), Fruchtschalen (Bergamotte, Zitrone, Orangen), Wurzeln (Macis, Angelica, Sellerie, Kardamon, Costus, Iris, Calmus), Hölzern (Pinien-, Sandel-, Guajak-, Zedern-, Rosenholz), Kräutern und Gräsern (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone, $\alpha$-Isomethylionon und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labolanumöl und Lavandinöl. Vorzugsweise werden Bergamotteöl, Dihydromyrcenol, Lilial, Lyral, Citronellol, Phenylethylalkohol, $\alpha$-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, $\beta$-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilllat, Irotyl und Floramat allein oder in Mischungen, eingesetzt.

Als **Aromen** kommen beispielsweise Pfefferminzöl, Krauseminzöl, Anisöl, Sternanisöl, Kümmelöl, Eukalyptusöl, Fenchelöl, Citronenöl, Wintergrünöl, Nelkenöl, Menthol und dergleichen in Frage.

Bei den bevorzugten oralen Zubereitungen kann es sich auch um **Kaugummis** handeln. Diese Produkte enthalten typischerweise eine wasserunlösliche und eine wasserlösliche Komponente.

Die wasserunlösliche Basis, die auch als "Gummibasis" bezeichnet wird, umfasst üblicherweise natürliche oder synthetische Elastomere, Harze, Fette und Öle, Weichmacher, Füllstoffe, Farbstoffe sowie gegebenenfalls Wachse. Der Anteil der Basis an der Gesamtzusammensetzung macht üblicherweise 5 bis 95, vorzugsweise 10 bis 50 und insbesondere 20 bis 35 Gew.-% aus. In einer typischen Ausgestaltungsform der Erfindung setzt sich die Basis aus 20 bis 60 Gew.-% synthetischen Elastomeren, 0 bis 30 Gew.-% natürlichen Elastomeren, 5 bis 55 Gew.-% Weichmachern, 4 bis 35 Gew.-% Füllstoffe und in untergeordneten Mengen Zusatzstoffe wie Farbstoffe, Antioxidantien und dergleichen zusammen, mit der Maßgabe, dass sie allenfalls in geringen Mengen wasserlöslich sind.

Als geeignete synthetische Elastomere kommen beispielsweise Polyisobutylene mit durchschnittlichen Molekulargewichten (nach GPC) von 10.000 bis 100.000 und vorzugsweise 50.000 bis 80.000, Isobutylen-Isopren-Copolymere ("Butyl Elastomere"), Styrol-Butadien-Copolymere (Styrol:Butadien-Verhältnis z.B. 1: 3 bis 3: 1), Polyvinylacetate mit durchschnittlichen Molekulargewichten (nach GPC) von 2.000 bis 90.000 und vorzugsweise 10.000 bis 65.000, Polyisoprene, Polyethylen, Vinylacetat-Vinyllaurat-Copolymere und deren Gemische. Beispiele für geeignete natürliche Elastomere sind Kautschuks wie etwa geräucherter oder flüssiger Latex oder Guayule sowie natürliche Gummistoffe wie Jelutong, Lechi caspi, Perillo, Sorva, Massaranduba balata, Massaranduba chocolate, Nispero, Rosindinba, Chicle,

Gutta hang 1kang sowie deren Gemische. Die Auswahl der synthetischen und natürlichen Elastomere und deren Mischungsverhältnisse richtet sich im Wesentlichen danach, ob mit den Kaugummis Blasen erzeugt werden sollen ("bubble gums") oder nicht. Vorzugsweise werden Elastomergemische eingesetzt, die Jelutong, Chicle, Sorva und Massaranduba enthalten.

In den meisten Fällen erweisen sich die Elastomere in der Verarbeitung als zu hart oder zu wenig verformbar, so dass es sich als vorteilhaft erwiesen hat, spezielle Weichmacher mitzuverwenden, die natürlich insbesondere auch alle Anforderungen an die Zulassung als Nahrungsmittelzusatzstoffe erfüllen müssen. In dieser Hinsicht kommen vor allem Ester von Harzsäuren in Betracht, beispielsweise Ester von niederen aliphatischen Alkoholen oder Polyolen mit ganz oder teilweise gehärteten, monomeren oder oligomeren Harzsäuren. Insbesondere werden für diesen Zweck die Methyl-, Glycerin-, oder Pentareythritester sowie deren Gemische eingesetzt. Alternativ kommen auch Terpenharze in Betracht, die sich von alpha-Pinen, beta-Pinen, delta-Limonen oder deren Gemischen ableiten können.

Als Füllstoffe oder Texturiermittel kommen Magnesium- oder Calciumcarbonat, gemahlener Bimsstein, Silicate, speziell Magnesium- oder Aluminiumsilicate, Tone, Aluminiumoxide. Talkum, Titandioxid, Mono-, Di- und Tricalciumphosphat sowie Cellulosepolymere.

Geeignete Emulgatoren sind Talg, gehärteter Talg, gehärtete oder teilweise gehärtete pflanzliche Öle, Kakaobutter, Partialglyceride, Lecithin, Triacetin und gesättigte oder ungesättigte Fettsäuren mit 6 bis 22 und vorzugsweise 12 bis 18 Kohlenstoffatomen sowie deren Gemische.

Als Farbstoffe und Weißungsmittel kommen beispielsweise die für die Färbung von Lebensmitteln zugelassenen FD und C-Typen, Pflanzen- und Fruchtextrakte sowie Titandioxid in Frage.

Die Basismassen können Wachse enthalten oder wachsfrei sein; Beispiele für wachsfreie Zusammensetzungen finden sich unter anderem in der Patentschrift US 5,286,500, auf deren Inhalt hiermit ausdrücklich Bezug genommen wird.

Zusätzlich zu der wasserunlöslichen Gummibasis enthalten Kaugummizubereitungen regelmäßig einen wasserlöslichen Anteil, der beispielsweise von Softener, Süßstoffen, Füllstoffen, Geschmacksstoffen, Geschmacksverstärkern, Emulgatoren, Farbstoffen, Säuerungsmitteln, Antioxidantien und dergleichen gebildet werden, hier mit der Maßgabe, dass die Bestandteile eine wenigstens hinreichende Wasserlöslichkeit besitzen. In Abhängigkeit der Wasserlöslichkeit der speziellen Vertreter können demnach einzelne Bestandteile sowohl der wasserunlöslichen wie auch der wasserlöslichen Phase angehören. Es ist jedoch auch möglich, Kombinationen beispielsweise eines wasserlöslichen und eines wasserunlöslichen Emulgators einzusetzen, wobei sich die einzelnen Vertreter, dann in unterschiedlichen Phasen befinden. Üblicherweise macht der wasserunlösliche Anteil 5 bis 95 und vorzugsweise 20 bis 80 Gew.-% der Zubereitung aus.

Wasserlösliche Softener oder Plastifiziermittel werden den Kaugummizusammensetzungen hinzugegeben um die Kaubarkeit und das Kaugefühl zu verbessern und sind in den Mischungen typischerweise in Mengen von 0,5 bis 15 Gew.-% zugegen. Typische Beispiele sind Glycerin, Lecithin sowie wässrige Lösungen von Sorbitol, gehärteten Stärkehydrolysaten oder Kornsirup.

Als zusätzliche Süßstoffe neben der kristallinen Allulose kommen sowohl zuckerhaltige wie zuckerfreie Verbindungen in Frage, die in Mengen von 5 bis 95, vorzugsweise 20 bis 80 und insbesondere 30 bis 60 Gew.-% bezogen auf die Kaugummizusammensetzung eingesetzt werden. Typische Saccharose-Süssstoffe sind Sucrose, Dextrose, Maltose, Dextrin, getrockneter Invertzucker, Fructose, Levulose, Galactose, Kornsirup sowie deren Gemische. Als Zuckerersatzstoffe kommen Sorbitol, Mannitol, Xylitol, gehärtete Stärkehydrolysate, Maltitol und deren Gemische in Frage. Weiterhin kommen als Zusatzstoffe auch sogenannte HIAS ("High Intensity Articifical Sweeteners") in Betracht, wie beispielsweise Sucralose, Aspartam, Acesulfamsalze, Alitam, Saccharose und Saccharosesalze, Cyclamsäure und deren Salze, Glycyrrhizine, Dihydrochalcone, Thaumatin, Monellin und dergleichen alleine oder in Abmischungen. Besonders wirksam sind auch die hydrophoben HIAS, die Gegenstand der internationalen Patentanmeldung WO 2002 091849 A1 (Wrigleys) sowie Stevia Extrakte und deren aktiven Bestandteile, insbesondere Ribeaudiosid A sind. Die Einsatzmenge dieser Stoffe hängt in erster Linie von ihrem Leistungsvermögen ab und liegt typischerweise im Bereich von 0,02 bis 8 Gew.-%.

Insbesondere für die Herstellung kalorienarmer Kaugummis eignen sich Füllstoffe wie beispielsweise Polydextrose, Raftilose, Rafitilin, Fructooligosaccharide (NutraFlora), Palatinoseoligosaaccharide, Guar Gum Hydrolysate (Sun Fiber) sowie Dextrine.

Die Auswahl an weiteren Geschmacksstoffen ist praktisch unbegrenzt und für das Wesen der Erfindung unkritisch. Üblicherweise liegt der Gesamtanteil aller Geschmacksstoffe bei 0,1 bis 15 und vorzugsweise 0,2 bis 5 Gew.-% bezogen auf die Kaugummizusammensetzung. Geeignete weitere Geschmacksstoffe stellen beispielsweise essentielle Öle, synthetische Aromen und dergleichen dar, wie etwa Anisöl, Sternanisöl, Kümmelöl, Eukalyptusöl, Fenchelöl, Citronenöl, Wintergrünöl, Nelkenöl, und dergleichen, wie sie auch beispielsweise in Mund- und Zahnpflegemittel Verwendung finden.

Die Kaugummis können des weiteren Hilfs- und Zusatzstoffe enthalten, die beispielsweise für die Zahnpflege, speziell zur Bekämpfung von Plaque und Gingivitis geeignet sind, wie z.B. Chlorhexidin, CPC oder Trichlosan. Weiter können pH-Regulatoren (z.B. Puffer oder Harnstoff), Wirkstoffe gegen Karies (z.B. Phosphate oder Fluoride), biogene

Wirkstoffe (Antikörper, Enzyme, Koffein, Pflanzenextrakte) enthalten sein, solange diese Stoffe für Nahrungsmittel zugelassen sind und nicht in unerwünschter Weise miteinander in Wechselwirkung treten.

## PHARMAZEUTISCHE MITTEL

Die geeigneten pharmazeutischen Mittel umfassen feste, tablettierte oder dragierte Wirkstoffe und Wirkstoffmischungen sowie flüssige Produkte. Pharmazeutische Produkte mit kristalliner Allulose zeichnen sich vor allem dadurch aus, dass sie nicht kariogen und damit auch für Diabetiker geeignet sind. In flüssigen Formulierungen findet keine Auskristallisation statt. Die Tablettierung gelingt einfacher, außerdem lassen sich Tabletten mit Allulose, wie beispielsweise "Aspirin Plus C" infolge der höheren Löslichkeit auch ohne Wasser einnehmen.

Im Sinne der Erfindung werden in einer ersten Ausführungsform (Lutsch-)Tabletten und Dragees, also feste Verabreichungsformen angesprochen, die einen oder mehrere pharmazeutische Wirkstoffe zusammen mit einem Tablettierungsmittel und gegebenenfalls einem Sprengmittel enthalten, deren Natur jedoch unkritisch ist.

Die Erfindung betrifft die überraschende Erkenntnis, dass der unangenehme, adstringierende und/oder metallische Nachgeschmack, der vielen pharmazeutischen Wirkstoffen (insbesondere den so genannten "non-steroidal anti-inflammatory drugs", NSAID) gemeinsam ist und deren Einnahme erschwert, durch die kristalline Allulose überdeckt und/oder maskiert wird. Geeignete Wirkstoffe, für die diese Erkenntnis zutrifft sind - ohne dabei abschließend zu sein - die folgenden:

(1) **ASPIRIN** (Acetylsalicylsäure)

(2) **MINOXIDIL** (6-piperidin-1-ylpyrimidin-2,4-diamin-3-oxid)

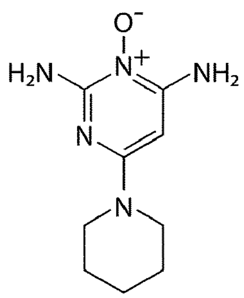

(3) **ERYTHROMICIN**

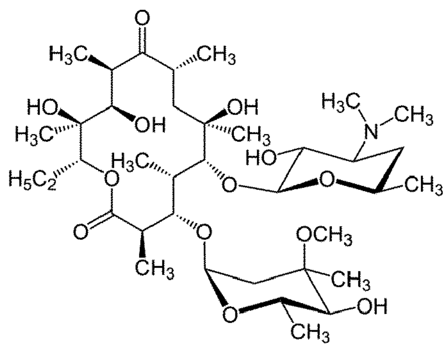

(4) **DIMETINDENE (FENISTIL)** (RS-dimethyl(2-(3-[pyridin-2-yl)ethyl]-1H-inden-2-yl)ethyl)amine)

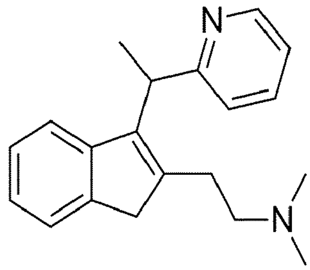

(5) **BETAMETHOSON** (8S,9R,10S.11S,13S,14S,16S,17R)-9-fluoro-11,17-(2-hydroxyacetyl)-10, 13,16-trimethyl-6,7,8,9,10,11,12,13,14,15,16,17-dodecahydro-3H-cyclopenta(alpha)-phenanthren-3-one

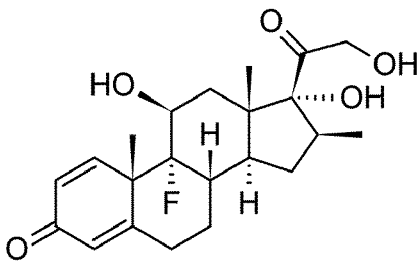

(6) **IBUPROFEN** (RS)-2-(4-(2-methylpropyl)phenyl)propanoic acid)

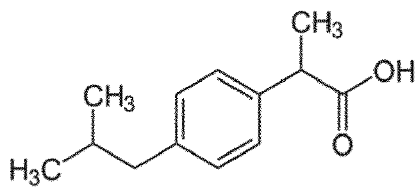

(7) **KETOPROFEN** (RS)2-(3-benzoylphenyl)-propionic acid)

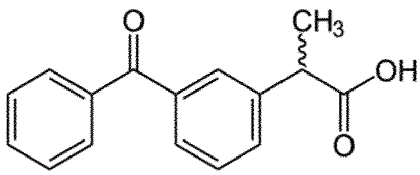

(8) **DICLOFENAC**

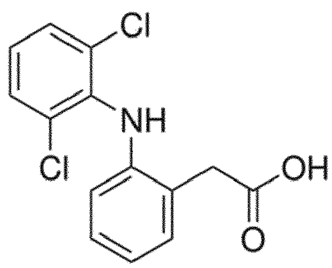

(9) **METRONIDAZOL** (2-(2-methyl-5-nitro-1H-imidazol-1-yl)ethanol)

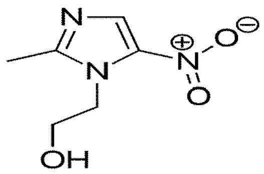

(10) **ACYCLOVIR** (2-Amino-1,9-dihydro-9-((2-hydroxyethoxy)methyl)-6H-Purin-6-one),

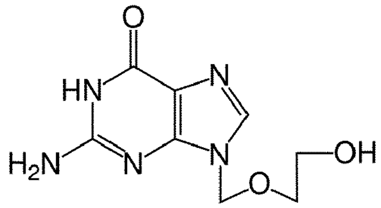

(11) **IMIQUIMOD** (3-(2-methylpropyl)-3,5,8-triazatricyclo[7.4.0.0.$^{2,6}$]trideca-1(9),2(6),4,7,10, 12- hexaen-7-amine

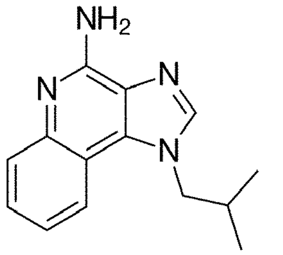

(12) **TERBINAFIN** [(2E)-6,6-dimethylhept-2-en-4-yn-1-yl](methyl)(naphthalen-1-ylmethyl)amine)

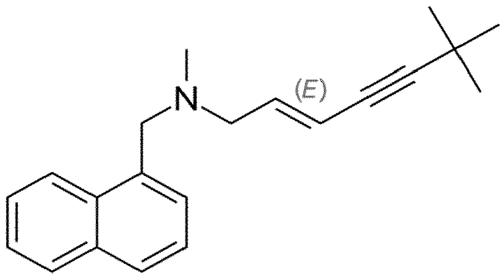

(13) **CICLOPYRAXOLAMIN** (6-cyclohexyl-1-hydroxy-4-methylpyridin-2(1H)-one)

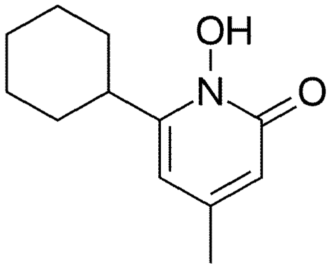

Neben den festen Zubereitungen sind auch flüssige Produkte mit einem Gehalt an Allulosekristallen Gegenstand der Erfindung, wie beispielsweise Hustensaft oder Sprayformulierungen.

## GEWERBLICHE ANWENDBARKEIT

Ein weiterer Gegenstand der vorliegenden Erfindung betrifft die Verwendung von Allulose in kristalliner Form zur Herstellung von oralen Zubereitungen, speziell Nahrungsmitteln, Mund- und Zahnpflegemitteln sowie pharmazeutischen Mitteln der oben genannten Art. Der guten Ordnung halber sei darauf hingewiesen, dass alle oben im Zusammenhang mit den konfektionierten Produkten genannten bevorzugten Stoffe, Stoffgemische, Einsatzmengen und dergleichen für die erfindungsgemäße Verwendung mitgelten, ohne dass dies noch einmal gesondert wiederholt werden muss-

## BEISPIELE

Die vorliegende Erfindung wird unter Bezugnahme auf die nachstehenden Beispiele leichter zu verstehen sein.
Jedoch dienen diese Beispiele lediglich zur Veranschaulichung der Erfindung und können nicht als einschränkend in Bezug auf den Schutzbereich der Erfindung ausgelegt werden. Alle Mengenangaben - wenn nicht anders angegeben - verstehen sich als Gew.-%.

## BEISPIEL 1

*Geschmackliche Eigenschaften von Softdrinks*

Verschiedene Stoffgemische auf Basis von Steviolglykosiden (Rebaudiosid A) mit und ohne Allulose-Kristallen wurden zur Herstellung von einfachen Softdrinks verwendet und die Produkte 48 h bei 20°C gelagert. Anschließend wurden die geschmacklichen Eigenschaften (Deskriptoren: Anfangssüße, Süßintensität, Zuckergeschmack/Mundfülle) von einem Panel bestehend aus 8 geschulten Testern auf einer Linien-Skala von 0 (nicht vorhanden) bis 10 (stark ausgeprägt) bewertet. Die Zusammensetzungen und Ergebnisse sind in der nachfolgenden **Tabelle 1** zusammengefasst. Die Ausführungen 1 und 2 sind erfindungsgemäß, die Beispiele V1 bis V3 dienen zum Vergleich. Das Beispiel C entspricht dem Standard, d.h. der geschmacklichen Beurteilung des Produktes ohne Zusatz von Stevia.

**Tabelle 1**

Geschmackliche Eigenschaften von Softdrinkformulierungen

| **Zusammensetzung** | **C** | **1** | **2** | **V1** | **V2** | **V3** |
|---|---|---|---|---|---|---|
| Allulose | - | 7,0 | 10,0 | - | - | - |
| Saccharose | 10,0 | - | - | 7,0 | 8,0 | 10,0 |
| Zitronensäure | 0.2 | 0.2 | 0.2 | 0.2 | 0.2 | 0.2 |
| Rebaudiosid A | - | 0,0045 | 0,0045 | 0,0045 | 0,0045 | 0,0045 |
| Zitronenaroma | 0,1 | 0,1 | 0,1 | 0,1 | 0,1 | 0,1 |
| Wasser | Ad 100 | | | | | |
| ***Geschmackliche Beurteilung*** | | | | | | |
| Anfangssüße | 3,0 | 5,5 | 5,6 | 3,2 | 3,3 | 4,2 |
| Süßintensität | 3,1 | 6,3 | 6,4 | 3,5 | 3,8 | 4,5 |
| Zuckergeschmack und Mundfülle | 3,0 | 5,9 | 5,5 | 3,0 | 3,3 | 3,5 |
| Bitterkeit beim Nachgeschmack | 0,3 | 0,2 | 0,2 | 0,5 | 0,4 | 0,2 |

Die beiden erfindungsgemäßen Zubereitungen zeigen sowohl gegenüber dem Standard als auch den Stevia-haltigen Zubereitungen mit Sucrose Vorteile bezüglich Anfangssüße, Süßintensität, Zuckergeschmack, Mundfülle und Bitterkeit beim Nachgeschmack.
Im Folgenden wird die Erfindung an Hand weiterer Formulierungsbeispiele illustriert. Bei der Allulose handelt es sich um ein kristallines Produkt, welches die bevorzugten Auswahlregeln zur Partikelgrößenverteilung - wie oben beschrieben - erfüllt und von der Savanna Ingredients GmbH erhältlich ist.

## FORMULIERUNGSBEISPIEL 1

**Erfrischungsgetränke**

| Zusammensetzung | 1.1 | 1.2 | 1.3 | 1.4 | 1.5 | 1.6 | 1.7 |
|---|---|---|---|---|---|---|---|
| Allulose | 10,0 | 10,0 | 7,0 | - | 10,0 | 8,0 | 7,0 |
| Rebaudiosid A | 0,0045 | 0,005 | 0,003 | 0,01 | 0,0045 | 0,0025 | 0,001 |
| Zitronensäure | 0,15 | 0,15 | 0,06 | 0,15 | 0,15 | 0,15 | 0,15 |
| Phosphorsäure | - | - | 0,07 | - | - | - | - |
| Zuckercouleur | - | - | 0,14 | - | - | - | - |
| Koffein | - | - | 0,01 | - | - | - | - |
| Zitrusaroma | 0,1 | 0,05 | - | 0,1 | - | 0,1 | 0,1 |
| Limonenaroma | - | 0,05 | - | - | 0,1 | - | - |
| Getränkeemulsion Typ "Cola" | - | - | 0,05 | - | - | - | - |
| Wasser | ad 100 | | | | | | |

## FORMULIERUNGSBEISPIEL 2

**Eistee**

| Zusammensetzung | 2.1 | 2.2 |
|---|---|---|
| Schwarztee-Extrakt | 1,4 | 1,4 |
| Wasser | ad 100 | |
| Aromazubereitung (Typ Pfirsich) | 0,65 | 0,65 |
| Zucker | 3,5 | 3,0 |
| Zitronensäure (kristallin) | 1,2 | 1,2 |
| Ascorbinsäure | 0,2 | 0,2 |
| Allulose | 3,5 | 4,0 |

## FORMULIERUNGSBEISPIEL 3

**Tee Instantgetränk**

| Zusammensetzung | 3.1 | 3.2 | 3.3 |
|---|---|---|---|
| schwarzer Tee, Ceylon, Blattware | 94,00 | - | - |
| grüner Tee, China, Blattware | - | 92,0 | - |
| Mate Tee, Peru, Blattware | - | - | 95,0 |
| Allulose | 6,0 | 8,0 | 5,0 |

## FORMULIERUNGSBEISPIEL 4

**Cappuccino Instantgetränk**

| Zusammensetzung | 4.1 | 4.2 |
|---|---|---|
| Kaffee-Extrakt, sprühgetrocknet | 14,0 | 16,0 |
| Zucker | 25,0 | 20,0 |
| Allulose | 6,0 | 11,0 |
| Fettpulver | 18,2 | 18,2 |
| Kaffeeweißer, schäumend | ad100 | |
| Hydrokolloide/ Emulgatoren | 1,8 | 1,8 |
| Lactose | 4,7 | 4,7 |

## FORMULIERUNGSBEISPIEL 5

**Bittere Schokolade**

| Zusammensetzung | 5.1 | 5.2 |
|---|---|---|
| Kakaomasse | ad 100 | |
| Kakaobutter | 11,70 | 11,70 |
| Zucker | 21,00 | 15,00 |
| Allulose | 9,00 | 15,00 |
| Vollmilch | 3,00 | 3,00 |
| Lezithin | 0,2 | 0,2 |
| Vanillin | 0,035 | 0,035 |

## FORMULIERUNGSBEISPIEL 6

**Fruchtgummis**

| Zusammensetzung | 6.1 | 6.2 |
|---|---|---|
| Allulose | 34,50 | 8,20 |
| Rebaudiosid A | 0,003 | 0,003 |
| Iso Sirup C* Tru Sweet 01750 (Cerestar GmbH) | 1,50 | 2,10 |
| Gelatine 240 Bloom | 8,20 | 9,40 |
| Polydextrose (Litesse® Ultra, Danisco Cultor GmbH) | - | 24,40 |
| Farbstoff | 0,01 | 0,01 |
| Zitrusaroma | 0,20 | - |
| Kirscharoma | - | 0,10 |
| Wasser | ad 100 | |

## FORMULIERUNGSBEISPIEL 7

**Zuckerfreie Lutschbonbons**

| Zusammensetzung | 7.1 | 7.2 | 7.3 |
|---|---|---|---|
| Wasser | 2,24 | 2,24 | 2,24 |
| Isomalt | | ad 100 | |
| Allulose | 2,40 | 2,60 | 3,0 |
| Sucralose | 0,03 | 0,03 | 0,03 |
| Acesulfam K | 0,050 | 0,050 | 0,050 |
| Zitronensäure | 0,050 | 0,050 | 0,050 |

## FORMULIERUNGSBEISPIEL 8

**Zahnpasten**

| Zusammensetzung | 8.1 | 8.2 | 8.3 |
|---|---|---|---|
| Gum base | | ad 100 | |
| Sorbit, gepulvert | 40,00 | 40,00 | 40,00 |
| Isomalt, gepulvert | 9,50 | 9,50 | 9,50 |
| Xylit | 2,00 | 1,00 | 1,00 |
| Mannit D | 2,00 | 3,00 | 2,00 |
| Allulose | 1,00 | 1,00 | 1,00 |
| Acesulfam K | 0,10 | 0,10 | 0,10 |
| Emulgum / Plastifizierungsmittel | 0,30 | 0,30 | 0,30 |
| Sorbit (70% Wasser) | 13,00 | 13,00 | 13,00 |
| Glycerin | 1,00 | 1,00 | 1,00 |

## FORMULIERUNGSBEISPIEL 9

**Zahnpasten**

| Zusammensetzung | 9.1 | 9.2 | 9.3 |
|---|---|---|---|
| Entionisiertes Wasser | 36,39 | 36,39 | 36,39 |
| Glycerin | 20,00 | 20,00 | 20,00 |
| Solbrol M (Natriumsalz) | 0,15 | 0,15 | 0,15 |
| Natriummonofluorphosphat | 0,76 | 0,76 | 0,76 |
| Allulose | 0,20 | 0,25 | 0,30 |
| Dicalciumphosphat-Dihydrat | 36,00 | 36,00 | 36,00 |
| Aerosil 200 | 3,00 | 3,00 | 3,00 |
| Natriumcarboxymethylcellulose | 1,20 | 1,20 | 1,20 |
| Natriumlaurylsulfat | 1,30 | 1,30 | 1,30 |

## FORMULIERUNGSBEISPIEL 10

**Mundwasserkonzentrat**

| Zusammensetzung | 10.1 | 10.2 | 10.3 |
|---|---|---|---|
| Ethanol 96% | 42,00 | 42,00 | 42,00 |
| Cremophor RH 455 | 5,00 | 5,00 | 5,00 |
| Entionisiertes Wasser | 50,67 | 50,67 | 50,67 |
| Allantoin | 0,20 | 0,20 | 0,20 |
| Allulose | 0,10 | 0,20 | 0,30 |
| Colour L-Blue 5000 (1% in Wasser) | 0,03 | 0,03 | 0,03 |
| Aroma | 2,00 | 2,00 | 2,00 |

## FORMULIERUNGSBEISPIEL 11

**Kaugummi**

| Zusammensetzung | 11.1 | 11.2 |
|---|---|---|
| Kaugummibase, Company "Jagum T" | 30,00 | 30,00 |
| Sorbit, pulverisiert | 39,00 | 39,00 |
| Isomalt® (Palatinit GmbH) | 9,50 | 9,50 |
| Allulose | 2,00 | 2,00 |
| Mannit | 3,00 | 3,00 |
| Aspartam® | 0,10 | 0,10 |
| Acesulfam® K | 0,10 | 0,10 |
| Emulgum® (Colloides Naturels, Inc.) | 0,30 | 0,30 |
| Sorbitol, 70% | 14,00 | 14,00 |
| Glycerin | 1,00 | 1,00 |
| Minzaroma | 1,00 | - |
| Tutti-Frutti-Aroma | - | 2,00 |

## FORMULIERUNGSBEISPIEL 12

**Gelatinekapsel**

| Zusammensetzung | 12.1 | 12.2 | 12.2 |
|---|---|---|---|
| **Gelatinehülle:** | | | |
| Glycerin | 2,014 | 2,014 | 2,014 |
| Gelatine 240 Bloom | 7,91 | 7,91 | 7,91 |
| Allulose | 0,065 | 0,085 | 0,10 |
| Allura-Rot | 0,006 | - | 0,011 |

(fortgesetzt)

| Zusammensetzung | 12.1 | 12.2 | 12.2 |
|---|---|---|---|
| **Gelatinehülle:** | | | |
| Brillantblau | 0,005 | 0,011 | - |
| **Kernzusammensetzung:** | | | |
| Pflanzenöl-Triglycerid (Kokosöl - Fraktion) | | ad 100 | |
| Orangen-Aroma | 10,0 | - | - |
| Pfefferminz-Aroma | - | 20,0 | - |
| Kirsch-Aroma | - | - | 28,65 |
| Rebaudiosid A 98 % | 0,05 | 0,05 | - |
| 2-Hydroxypropylmenthylcarbonat | 0,33 | 0,20 | - |
| 2-Hydroxyethylmenthylcarbonat | - | 0,20 | 1,00 |
| (1R,3R,4S) Menthyl-3-carbonsäure-N-ethylamid (WS-3) | - | 0,55 | - |
| (-)-Menthyllactat (Frescolat ML) | - | 0,30 | - |
| Vanillin | 0,07 | - | 0,10 |

## FORMULIERUNGSBEISPIEL 13

**Magerjoghurt**

| Zusammensetzung | 13.1 | 13.2 | 13.3 | 13.4 |
|---|---|---|---|---|
| Allulose | 8,0 | 10,0 | 8,0 | 10,0 |
| Rebaudiosid A | - | - | 0,025 | 0,025 |
| Saccharin | - | 0,3 | 0,3 | 0,3 |
| Sauerkirsch-Extrakt | 0,2 | 0,1 | 0,2 | 0,2 |
| Joghurt, 0,1 % Fett | | ad 100 | | |

## FORMULIERUNGSBEISPIEL 14

**Pudding**

| Zusammensetzung | 14.1 | 14.2 | 14.3 |
|---|---|---|---|
| Maisstärke | 38 g | 38 g | 38 g |
| Allulose | 38 g | 28 g | 19 g |
| Zucker | - | 10 g | 19 g |
| Rebaudiosid A | - | 0,05 g | 0,08 g |
| Vanille-Aroma (Symrise) | 0,2 g | 0,2 g | 0,2 g |
| Chinolingelb | 0,02 g | 0,02 g | 0,02 g |
| Milch | 500 ml | 500 ml | 500 ml |

FORMULIERUNGSBEISPIEL 15

**Brownies**

| Zusammensetzung | 15.1 | 15.2 | 15.3 |
|---|---|---|---|
| Vollei | 240,0 g | 240,0 g | 240,0 g |
| Salz | 2,0 g | 2,0 g | 2,0 g |
| Wasser | - | 40,0 g | 60,0 g |
| Allulose, kristallin | 260,0 g | 260,0 g | 400,0 g |
| Allulose-Sirup, flüssig | 200,0 g | - | - |
| Allulose-Sirup, fest | - | 160,0 g | - |
| Butter | 230,0 g | 230,0 g | 230,0 g |
| Mehl Typ 405 | 70,0 g | 70,0 g | 70,0 g |
| Kakaopulver | 110,0 g | 110,0 g | 110,0 g |
| Aroma (Vanille) | 5,0 g | 5,0 g | 5,0 g |

## Patentansprüche

1. Orale Zubereitungen, enthaltend Allulose in kristalliner Form.

2. Zubereitungen nach Anspruch 1, **dadurch gekennzeichnet, dass** die kristalline Allulose einen Fructosegehalt von maximal 1,5 Gew.-% aufweist.

3. Zubereitungen nach Anspruch 2, **dadurch gekennzeichnet, dass** die kristalline Allulose einen Aschegehalt von weniger als 0,1 Gew.-% aufweist.

4. Zubereitungen nach mindestens einem der Ansprüche 1 bis 4, **dadurch gekennzeichnet, dass** es sich um Nahrungsmittel, Mund- und Zahnpflegemittel oder pharmazeutische Mittel handelt.

5. Zubereitungen nach Anspruch 4, **dadurch gekennzeichnet, dass** die Nahrungsmittel ausgewählt sind aus der Gruppe, die gebildet wird von Getränken, Backwaren, Getreideprodukten, Knabberartikeln, Süßwaren, Milchprodukten, Fruchtzubereitungen, Würzmitteln, Gemüsezubereitungen, Fleischwaren, Gewürzen und Gewürzmischungen.

6. Zubereitungen nach Anspruch 5, **dadurch gekennzeichnet, dass** die Getränke ausgewählt sind aus der Gruppe, die gebildet wird von Kaffee, Tee, Eistee, Wein, weinhaltigen Getränke, Bier, bierhaltigen Getränken, Likören, Schnäpsen, Weinbränden, (karbonisierten) fruchthaltigen Limonaden, (karbonisierten) isotonischen Getränken, mit Geschmacksstoffen angereichertem Mineralwasser, (karbonisierten) Erfrischungsgetränken, Nektaren, Schorlen, Obst- und Gemüsesäften, Frucht- oder Gemüsesaftzubereitungen, Instant-Kakao-Getränken, Instant-Tee-Getränken, Instant-Kaffeegetränken und Instant-Fruchtgetränken.

7. Zubereitungen nach Anspruch 5, **dadurch gekennzeichnet, dass** die Backwaren, Getreideprodukte und Knabberartikel ausgewählt sind aus der Gruppe, die gebildet wird von Brot, Trockenkeksen, Kuchen, Muffins, Donuts, sonstigem Gebäck, gebackenen oder frittierten Kartoffelchips oder Kartoffelteigprodukten, Extrudaten auf Mais- oder Erdnussbasis, Frühstückscerealien, Müsliriegeln und vorgegarten Fertigreis-Produkten.

8. Zubereitungen nach Anspruch 5, **dadurch gekennzeichnet, dass** die Süßwaren und Fruchtzubereitungen ausgewählt sind aus der Gruppe, die gebildet wird von Schokoladen, Schokoladenriegelprodukten, sonstige Riegelprodukten, Fruchtgummis, Hart- und Weichkaramellen, Kaugummis, Tabletten, Mints, Überzügen (Fondants), Konfitüren, Fruchteis, Fruchtsoßen und Fruchtfüllungen.

9. Zubereitungen nach Anspruch 5, **dadurch gekennzeichnet, dass** die Milchprodukte ausgewählt sind aus der Gruppe, die gebildet wird von Milchgetränken, Buttermilchgetränken, Milcheis, Joghurt, Kefir, Frischkäse, Weichkäse, Hartkäse, Trockenmilchpulvern, Molke, Molkegetränken, Butter, Buttermilch, teilweise oder ganz hydrolysierte Milchprotein-haltigen Produkten, Produkte aus Sojaprotein oder anderen Sojabohnen-Fraktionen, Fruchtgetränken mit Sojaprotein, Sojalecithin-haltige Zubereitungen, fermentierte Produkte wie Tofu oder Tempe oder daraus gefertigte Produkte) und Produkten aus anderen pflanzlichen Proteinquellen.

10. Zubereitungen nach Anspruch 5, **dadurch gekennzeichnet, dass** die Würzmittel und Gemüsezubereitungen ausgewählt sind aus der Gruppe, die gebildet wird von Ketchup, Soßen, Trockengemüsen, Tiefkühlgemüsen, vorgegarten Gemüsen, eingekochten Gemüsen, Mayonnaise, Remoulade, Dressings, Fertiggerichten, Trockensuppen, Instant-Suppen, vorgegarte Suppen, Gewürzen, Würzmischungen und Aufstreuwürzen (englisch: Seasonings).

11. Zubereitungen nach Anspruch 5, **dadurch gekennzeichnet, dass** die Fleischprodukte ausgewählt sind aus der Gruppe, die gebildet wird von Schinken, Frischwurst- oder Rohwurstzubereitungen, und gewürzten oder marinierten Frisch- oder Pökelfleischprodukten.

12. Zubereitungen nach Anspruch 4, **dadurch gekennzeichnet, dass** die Gewürze und Gewürzmischungen ausgewählt sind aus der Gruppe, die gebildet wird von Süßkraftverstärkern, Geschmacksverstärkern und Maskierungsmitteln.

13. Zubereitungen nach Anspruch 4, **dadurch gekennzeichnet, dass** die Mund-und Zahnpflegemittel ausgewählt sind aus der Gruppe, die gebildet wird von Zahnpasten, Mundwässern und Kaugummis.

14. Zubereitungen nach Anspruch 4, **dadurch gekennzeichnet, dass** die pharmazeutischen Mittel ausgewählt sind aus der Gruppe, die gebildet wird von festen, tablettierten oder dragierten Wirkstoffen und Wirkstoffmischungen sowie flüssigen Produkten.

15. Zubereitungen nach mindestens einem der Ansprüche 1 bis 14, **dadurch gekennzeichnet, dass** sie die kristalline Allulose in Mengen von etwa 0,1 bis etwa 50 Gew.-% enthalten.

16. Verwendung von Allulose in kristalliner Form zur Herstellung von oralen Zubereitungen.

Europäisches Patentamt
European Patent Office
Office européen des brevets

# EUROPÄISCHER RECHERCHENBERICHT

Nummer der Anmeldung
EP 20 15 6808

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | US 2016/302463 A1 (WOODYER RYAN D [US] ET AL) 20. Oktober 2016 (2016-10-20)<br>* Absätze [0084], [0085]; Ansprüche 1-5 *<br>----- | 1-10,12, 13,15,16 | INV.<br>A23L2/60<br>A23L27/30<br>A23L29/30<br>A61Q11/00 |
| X | US 2017/079313 A1 (WOODYER RYAN D [US] ET AL) 23. März 2017 (2017-03-23)<br>* Absätze [0066], [0082], [0098] *<br>----- | 1,4-12, 14,16 | |
| X | KR 2019 0003307 A (SAMYANG CORP [KR]) 9. Januar 2019 (2019-01-09)<br>* Zusammenfassung *<br>& EP 3 647 317 A1 (SAMYANG CORP [KR]) 6. Mai 2020 (2020-05-06)<br>* Absätze [0007], [0013], [0026] *<br>----- | 1,16 | |
| X | US 2017/223980 A1 (UNGUREANU IOANA MARIA [US] ET AL) 10. August 2017 (2017-08-10)<br>* Absatz [0026]; Anspruch 1 *<br>----- | 1,16 | |
| X | US 2014/271746 A1 (WOODYER RYAN D [US] ET AL) 18. September 2014 (2014-09-18)<br>* Ansprüche 1,7-14 *<br>----- | 1,16 | RECHERCHIERTE SACHGEBIETE (IPC) |
| X | US 10 342 247 B2 (ROQUETTE FRERES [FR]) 9. Juli 2019 (2019-07-09)<br>* Ansprüche 1,5,12 *<br>----- | 1,16 | A23L<br>A61Q |

Der vorliegende Recherchenbericht wurde für alle Patentansprüche erstellt

1

| Recherchenort | Abschlußdatum der Recherche | Prüfer |
|---|---|---|
| Den Haag | 6. August 2020 | Galleiske, Anke |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

**ANHANG ZUM EUROPÄISCHEN RECHERCHENBERICHT ÜBER DIE EUROPÄISCHE PATENTANMELDUNG NR.** EP 20 15 6808

In diesem Anhang sind die Mitglieder der Patentfamilien der im obengenannten europäischen Recherchenbericht angeführten Patentdokumente angegeben. Die Angaben über die Familienmitglieder entsprechen dem Stand der Datei des Europäischen Patentamts am Diese Angaben dienen nur zur Unterrichtung und erfolgen ohne Gewähr.

06-08-2020

| Im Recherchenbericht angeführtes Patentdokument | Datum der Veröffentlichung | Mitglied(er) der Patentfamilie | Datum der Veröffentlichung |
|---|---|---|---|
| US 2016302463 A1 | 20-10-2016 | BR 112016011595 A2 | 12-09-2017 |
| | | CA 2931320 A1 | 28-05-2015 |
| | | CL 2016001222 A1 | 02-12-2016 |
| | | JP 2017500019 A | 05-01-2017 |
| | | KR 20160089551 A | 27-07-2016 |
| | | US 2016302463 A1 | 20-10-2016 |
| | | WO 2015075473 A1 | 28-05-2015 |
| US 2017079313 A1 | 23-03-2017 | AR 100522 A1 | 12-10-2016 |
| | | AU 2015263073 A1 | 08-12-2016 |
| | | AU 2019201408 A1 | 28-03-2019 |
| | | CA 2949595 A1 | 26-11-2015 |
| | | CL 2016002955 A1 | 12-05-2017 |
| | | CN 106659203 A | 10-05-2017 |
| | | EP 3154378 A1 | 19-04-2017 |
| | | GB 2526383 A | 25-11-2015 |
| | | JP 6728066 B2 | 22-07-2020 |
| | | JP 2017515487 A | 15-06-2017 |
| | | KR 20170009894 A | 25-01-2017 |
| | | US 2017079313 A1 | 23-03-2017 |
| | | WO 2015177522 A1 | 26-11-2015 |
| KR 20190003307 A | 09-01-2019 | CN 110869379 A | 06-03-2020 |
| | | EP 3647317 A1 | 06-05-2020 |
| | | KR 20190003307 A | 09-01-2019 |
| | | TW 201904446 A | 01-02-2019 |
| | | US 2020196648 A1 | 25-06-2020 |
| US 2017223980 A1 | 10-08-2017 | BR 112018007293 A2 | 23-10-2018 |
| | | CA 3002583 A1 | 27-04-2017 |
| | | CN 108135223 A | 08-06-2018 |
| | | EP 3364775 A1 | 29-08-2018 |
| | | JP 2018531030 A | 25-10-2018 |
| | | SG 11201802875S A | 30-05-2018 |
| | | US 2017223980 A1 | 10-08-2017 |
| | | WO 2017068033 A1 | 27-04-2017 |
| US 2014271746 A1 | 18-09-2014 | AR 095475 A1 | 21-10-2015 |
| | | AU 2014229723 A1 | 22-10-2015 |
| | | BR 112015022306 A2 | 18-07-2017 |
| | | CA 2906893 A1 | 18-09-2014 |
| | | CN 105338833 A | 17-02-2016 |
| | | EP 2983510 A1 | 17-02-2016 |
| | | IL 241508 A | 29-08-2019 |
| | | JP 6457954 B2 | 23-01-2019 |
| | | JP 2016510987 A | 14-04-2016 |

EPO FORM P0461

Für nähere Einzelheiten zu diesem Anhang : siehe Amtsblatt des Europäischen Patentamts, Nr.12/82

## ANHANG ZUM EUROPÄISCHEN RECHERCHENBERICHT ÜBER DIE EUROPÄISCHE PATENTANMELDUNG NR.

EP 20 15 6808

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

06-08-2020

| Im Recherchenbericht angeführtes Patentdokument | Datum der Veröffentlichung | Mitglied(er) der Patentfamilie | Datum der Veröffentlichung |
|---|---|---|---|
| | | KR 20150130437 A | 23-11-2015 |
| | | MX 365386 B | 31-05-2019 |
| | | PL 2983510 T3 | 31-10-2018 |
| | | TW 201441245 A | 01-11-2014 |
| | | US 2014271746 A1 | 18-09-2014 |
| | | US 2016021917 A1 | 28-01-2016 |
| | | US 2017027206 A1 | 02-02-2017 |
| | | WO 2014140632 A1 | 18-09-2014 |
| US 10342247 B2 | 09-07-2019 | BR 112017001192 A2 | 21-11-2017 |
| | | CA 2955849 A1 | 28-01-2016 |
| | | CL 2017000136 A1 | 28-07-2017 |
| | | CN 106535661 A | 22-03-2017 |
| | | EP 3171710 A1 | 31-05-2017 |
| | | JP 2017526651 A | 14-09-2017 |
| | | KR 20170032899 A | 23-03-2017 |
| | | SG 11201700451Q A | 27-02-2017 |
| | | US 2018206538 A1 | 26-07-2018 |
| | | US 2019289888 A1 | 26-09-2019 |
| | | WO 2016012854 A1 | 28-01-2016 |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2018087261 A1
- WO 2016135358 A1
- WO 201820103 A1
- WO 2014186084 A1
- US 20020188019 A
- DE 102004041496
- WO 200610602 A
- WO 2005096841 A
- WO 2006003107 A
- EP 2006067120 W
- WO 2006058893 A
- WO 2007003527 A
- US 3488419 A
- DE 2224430 A1
- DE 2343196 A1
- US 5286500 A
- WO 2002091849 A1


### In der Beschreibung aufgeführte Nicht-Patentliteratur


- **H. ITOH et al.** *Journal of Fermentation Bioengeneering,* 1995, vol. 80 (1), 101-103
- **N. WAGNER et al.** *Organic Process Research Development,* 2012, vol. 16, 323-330
- **N. WAGNER et al.** *Chemical Engineering Science,* 2015, vol. 137, 423-435
- **N. WAGNER et al.** *Angewandte Chemie Int. Ed. Engl.,* 2015, vol. 54 (14), 4182-6
- **BOSSHART et al.** *Biotechnology Bioengineering,* 2016, vol. 113 (2), 349-58
- **N. WAGNER et al.** *Journal of Chromatography A,* 2015, vol. 1398, 47-56